# EUROPEAN PATENT SPECIFICATION

(11) **EP 3 594 200 B1**
(45) Date of publication and mention of the grant of the patent: **02.03.2022**
(21) Application number: 18764052.9
(22) Date of filing: 05.03.2018
(51) Int. Cl.: C07B 59/00, C07C 309/76, C07C 41/22, C07C 43/174, C07C 309/77, C07D 401/14, C07D 471/04, A61K 51/00, A61K 51/04

(54) **RADIOACTIVE FLUORINE-LABELED PRECURSOR COMPOUND, AND METHOD FOR PRODUCING RADIOACTIVE FLUORINE-LABELED COMPOUND USING SAME**
RADIOAKTIVE FLUORMARKIERTE VORLÄUFERVERBINDUNG UND VERFAHREN ZUR HERSTELLUNG EINER RADIOAKTIVEN FLUORMARKIERTEN VORLÄUFERVERBINDUNG DAMIT
COMPOSÉ PRÉCURSEUR MARQUÉ AU FLUOR RADIOACTIF, ET PROCÉDÉ DE PRODUCTION D'UN COMPOSÉ MARQUÉ AU FLUOR RADIOACTIF UTILISANT CE DERNIER

(30) Priority: 07.03.2017 JP 2017042783
(43) Date of publication of application: 15.01.2020
(73) Proprietor: Nihon Medi-Physics Co., Ltd., Tokyo 136-0075 (JP); Tokyo Institute of Technology, Tokyo 152-8550 (JP)
(72) Inventor: KIRIU, Masato, Tokyo 136-0075 (JP); ICHIKAWA, Hiroaki, Tokyo 136-0075 (JP); OKUMURA, Yuki, Tokyo 136-0075 (JP); TANAKA, Hiroshi, Tokyo 152-8550 (JP)
(74) Representative: TBK
(86) International application number: PCT/JP2018/008287
(87) International publication number: WO 2018/164043

(56) References cited:
- WO-A1-2007/135890
- WO-A1-2015/199205
- JP-A- 2007 526 916
- JP-A- 2011 516 517
- JP-A- 2012 509 887
- JP-A- 2012 532 833
- JP-A- 2017 052 713
- DATABASE CAS [Online] 8 August 2012 (2012-08-08), retrieved from STN Database accession no. 1387851-74-2
- CHO, C. et al.: "Nickel-Catalyzed Cross-Coupling of Neopentyl Arenesulfonates with Methyl and Primary Alkylmagnesium Bromides", Journal of Organic Chemistry, vol. 70, no. 4, 2005, pages 1482-1485, XP055315110, DOI: doi:10.1021/jo048300c
- ALBERT J. LEO: CHEMICAL REVIEWS, vol. 93, no. 4, 1993, pages 1281-1306,

## Description

### Technical Field

The present invention relates a novel radioactive fluorine-labeling precursor compound and a production method of a radioactive fluorine-labeled compound using the precursor compound.

### Background Art

Conventionally, a radioactive fluorine-labeling reaction is often performed by preparing a labeling precursor compound which is a compound having a leaving group bonded to a site to be fluorine-labeled of a target substrate and performing a nucleophilic substitution reaction in which radioactive fluoride ion F is allowed to react with the labeling precursor compound. In general, this reaction is performed by using a small amount of radioactive fluoride ion F with respect to a large amount of labeling precursor compound. Therefore, purification of the obtained radioactive fluorine-labeled compound is usually performed by separating a large amount of unreacted labeling precursor compound by a high-performance liquid chromatography (HPLC) method.

However, the HPLC method is cumbersome and takes time, and thus causes degradation of yield of the object compound in consideration of the half-life of radioactive fluorine of 110 minutes. As an alternative strategy requiring no HPLC purification, Patent Literatures 1 and 2 have proposed that a labeled compound is made easy to separate from another species without a compound M (purification site) by modifying a part of a leaving group of the labeling precursor compound with a compound M (purification site), and this compound is allowed to be used as a labeling precursor compound and to react with a nucleophilic agent such as radioactive fluoride ion F.

In addition, the present applicant has already filed a patent application for a labeling precursor compound and a labeling method using a novel leaving group different from the conventional leaving group (Patent Literature 3).

### Citation List

### Patent Literature

Patent Literature 1: WO 2009/127372 A
Patent Literature 2: WO 2011/006610 A
Patent Literature 3: JP 2017-52713 A
JP 2011 5165517 A and DATABASE CAS 8 August 2012 describe compounds having a structure corresponding to formula (2) of the present invention.

### Summary of Invention

The method described in Patent Literature 1 is based on the concept that an active group immobilized on a resin chemically acts on the purification site M of the precursor compound after the radioactive fluorination reaction. Therefore, there has been a problem in that radioactive fluorination rate is adversely affected, preparation of resins such as those having a specific active group introduced thereinto is required, or addition of further reaction conditions such as heating or addition of a reagent after the radioactive fluorination reaction is required.

The method described in Patent Literature 2 is based on the concept that a difference between logD of a labeling precursor compound and logD of a radioactive labeled compound is 1.5 or more, such that separation of the radioactive labeled compound is easily performed. However, there have been problems in that the type of leaving group disclosed in Patent Literature 2 is limited, designing in accordance with the characteristics of individual substrates is difficult, and poisonous chlorosulfonic acid needs to be used in synthesis. In addition, further improvement of purity of the separated radioactive labeled compound is required.

In addition, the method of Patent Literature 3 requires that the substrate is a compound having a neopentyl group, but does not focus on any application to a compound having no neopentyl group.

An object of the present invention is to provide a method which enables a leaving group to be flexibly designed, maintains a radioactive fluorination rate at the same degree as in conventional methods, and can separate and purify a radioactive fluorine-labeled compound from an unreacted precursor compound by a simple purification method after the radioactive fluorination reaction.

As a result of diligent research to solve the problems described above, the present inventors have found that a method which can maintain a radioactive fluorination rate at the same degree as in conventional methods and can separate and purify a radioactive fluorine-labeled compound from an unreacted precursor compound by a simple purification method after a radioactive fluorination reaction can be provided by introducing a hydrophobic amide tag into a benzene ring of a leaving group formed of a benzenesulfonyloxy group, thereby completing the present invention.

That is, according to an aspect of the present invention, there is provided a labeling precursor compound for a radioactive fluorine-labeled compound represented by the following general formula (1): 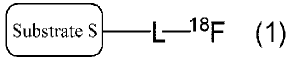 wherein S represents a substrate, and L represents a straight alkyl group having 1 to 6 carbon atoms, which may contain an ether group,
the labeling precursor compound being represented by the following general formula (2): 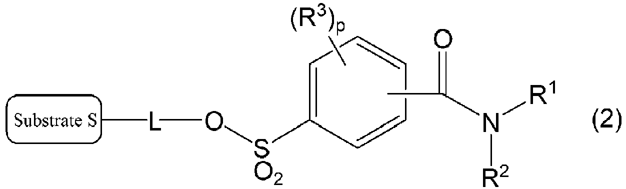 wherein S and L are the same as in the general formula (1), R¹ and R² each independently represent a straight or branched alkyl group having 1 to 30 carbon atoms, or a substituted or unsubstituted monocyclic or condensed polycyclic aryl group,
R³ each independently represent an alkyl group having 1 to 4 carbon atoms or an alkoxy group having 1 to 4 carbon atoms, and
p represents an integer of 0 to 4, characterized in that the radioactive fluorine-labeled compound represented by the general formula (1) has a clogP of -1.4 to 5.0, and a difference in clogP between the radioactive fluorine-labeled compound represented by the general formula (1) and the precursor compound represented by the general formula (2) is 2 or more.

In addition, according to another aspect of the present invention, there is provided a production method of a radioactive fluorine-labeled compound, the method including a step of allowing the aforementioned labeling precursor compound to react with [¹⁸F]fluoride ion to obtain the radioactive fluorine-labeled compound represented by the aforementioned general formula (1).

According to the present invention, since a compound represented by the general formula (2), that is, a compound in which a hydrophobic amide substituent is introduced into a benzene ring of a benzenesulfonyloxy group which is a leaving group, is used as a labeling precursor compound for a radioactive fluorine-labeling reaction, a radioactive fluorination rate is maintained at the same degree as in conventional methods, and a radioactive fluorine-labeled compound can be separated and purified from an unreacted precursor compound by a simple purification method after a radioactive fluorination reaction.

### Description of Embodiments

### 1. Radioactive fluorine-labeling precursor compound

A radioactive fluorine-labeling precursor compound of the present invention is a precursor compound for a radioactive fluorine-labeled compound represented by the general formula (1), and has a structure represented by the general formula (2). clogP(clogP₍₁₎) of the radioactive fluorine-labeled compound represented by the general formula (1) is -1.4 to 5.0 and preferably 2.0 to 5.0. The labeling precursor compound is designed such that a difference (clogP₍₂₎ - clogP₍₁₎) between clogP(clogP₍₁₎) of the radioactive fluorine-labeled compound represented by the general formula (1) and clogP(clogP₍₂₎) of the precursor compound represented by the general formula (2) is 2 or more, preferably 3 or more, more preferably 5 or more, and particularly preferably 8 or more. The upper limit thereof is not particularly limited, but the difference between clogPs (clogP₍₂₎ - clogP₍₁₎) is preferably 50 or less and is more practically 30 or less in consideration of solubility of the precursor compound in a reaction solution. By doing so, after the radioactive fluorine-labeling reaction, the unreacted precursor compound and the targeted radioactive fluorine-labeled compound can be easily separated from each other in a short time by simple column chromatography such as a reverse-phase cartridge column.

In the present invention, the alkyl group of R¹ and R² includes a straight or branched alkyl group having 1 to 30 carbon atoms among which the number of carbon atoms is preferably 4 to 24 and more preferably 8 to 18, and a straight alkyl group is preferable. The monocyclic aryl group of R¹ and R² includes a phenyl group, and the condensed polycyclic aryl group of R¹ and R² include a naphthyl group, an anthracenyl group, and the like. In the alkyl group and the monocyclic or condensed polycyclic aryl group, a hydrogen atom may be substituted by an alkyl group, an alkoxy group, a halogen atom, or the like. R¹ and R² may be the same or different, but are preferably the same group. A group represented by -CONR¹R² of the precursor compound of the present invention may be bonded to any of a metaposition, an ortho-position, and a para-position of the phenyl group, but is preferably bonded to a para-position of the phenyl group.

Note that, in the present specification, halogen means fluorine, chlorine, bromine, or iodine.

In the present invention, an example of the alkyl group of R³ includes a straight or branched alkyl group having 1 to 4 carbon atoms, and an example of the alkoxy group of R³ includes a straight or branched alkoxy group having 1 to 4 carbon atoms. In the precursor compound of the present invention, p represents an integer of 0 to 4, among which p is preferably 0 that is, a case where the phenyl group of the compound represented by the general formula (2) is substituted by no other substituents than -CONR¹R².

The radioactive fluorine-labeling precursor compound of the present invention is preferably one represented by the general formula (2) in which -CONR¹R² (R¹ and R² each independently preferably represent a straight alkyl group having 1 to 30 carbon atoms, or a substituted or unsubstituted condensed polycyclic aryl group) is bonded to a para-position, and p is 0.

In the present invention, L represents a straight alkyl group (linker) having 1 to 6 carbon atoms, which may contain an ether group. L can be, for example, a group represented by *-O(CH₂)ₙ-, *-(CH₂)ₙ-, or *-(OCH₂CH₂)ₘ-wherein n is an integer of 1 to 5, m is an integer of 1 to 3, and * represents a binding site to S.

In the present invention, S can be arbitrarily adopted as long as the compound represented by the general formula (1) is one used as a radiopharmaceutical. However, S can be, for example, a group represented by the following formula (S-1) or a group represented by the following formula (S-2).

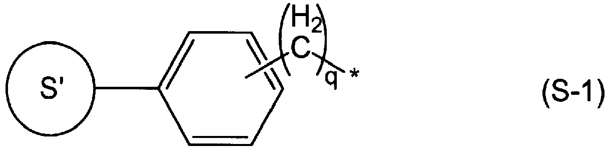

In the formula (S-1), S' is a part of S, q is 0 or 1, and the asterisk is a binding site to L.

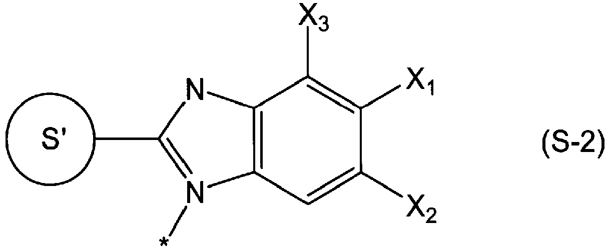

In formula (S-2), S' is a part of S, X₁ and X₃ each independently represent a hydrogen atom or a halogen atom, and X₂ represents a hydrogen atom, a halogen atom, or a nitrile group, but at least one of X₁, X₂, and X₃ is a halogen atom, and the asterisk is a binding site to L.

In the present invention, specific examples of the group represented by the formula (S-1) include groups represented by the following formulas (S-3), (S-4), (S-5), and (S-6). 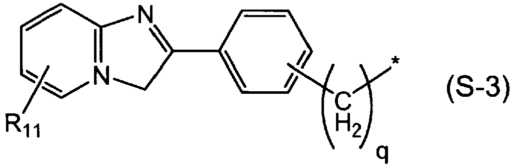 wherein q is the same as in the formula (S-1), R₁₁ is a halogen atom, and the asterisk represents a binding site to L. 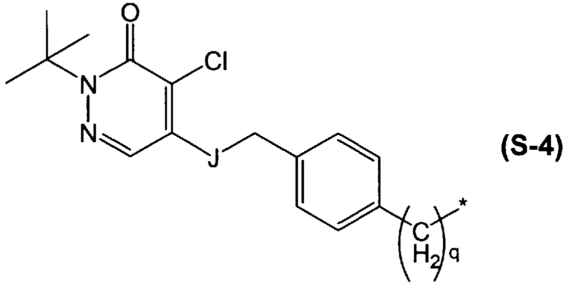 wherein q is the same as in the formula (S-1), J is O, S, NH, or NMe, the asterisk represents a binding site to L, and, here, Me represents a methyl group. 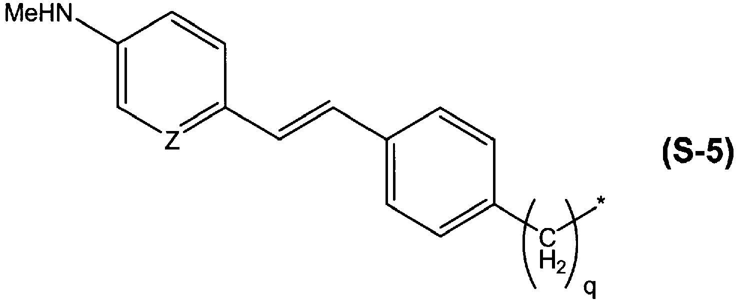 wherein q is the same as in the formula (S-1), Z is carbon or nitrogen, Me represents a methyl group, and the asterisk represents a binding site to L. 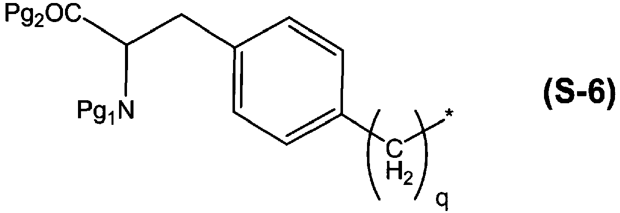 wherein q is the same as in the formula (S-1), Pg₁ represents a protecting group of an amino group, Pg₂ represents a protecting group of a carboxyl group, and the asterisk is a binding site to L.

In the formula (S-3), q is preferably 1. In addition, in a case where S is a group represented by formula (S-3), L is preferably a group presented by *-O(CH₂)ₙ- wherein * is a binding site to the formula (S-3), and n is an integer of 1 to 5 and preferably 2 to 4.

In the formula (S-4), q is preferably 0, and J is preferably O. In addition, in a case where S is a group represented by the formula (S-4), L is preferably a group presented by *-O(CH₂)ₙ- wherein * is a binding site to the formula (S-4), and n is an integer of 1 to 5 and preferably 2.

In the formula (S-5), q is preferably 0. In addition, in a case where S is a group represented by the formula (S-5), L is preferably a group presented by *-(OCH₂CH₂)ₘ- wherein * is a binding site to the formula (S-5), and m is an integer of 1 to 3 and preferably 3.

In the formula (S-6), q is preferably 0. In addition, in a case where S is a group represented by the formula (S-6), L is preferably a group presented by *-(CH₂)ₙ- wherein * is a binding site to the formula (S-6), and n is an integer of 1 to 5 and preferably 2.

In addition, in the present invention, a specific example of the group represented by the formula (S-2) includes a group represented by the following formula (S-7). 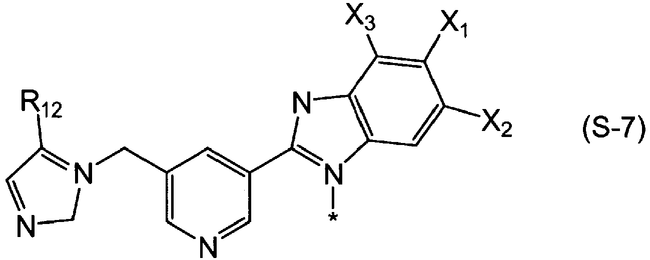 wherein X₁, X₂, and X₃ are the same as in the formula (S-2), R₁₂ represents a hydrogen atom, a halogen atom, or CO₂Rₐ, Rₐ represents an alkyl group having 1 to 10 carbon atoms, and the asterisk represents a binding site to L.

In the formula (S-7), R₁₂ is preferably a hydrogen atom, X₁ is preferably a hydrogen atom or a halogen atom, X₂ is preferably a halogen atom independently of X₁, and X₃ is preferably a hydrogen atom. In addition, in a case where S is a group represented by the formula (S-7), L is preferably a group represented by *-(CH₂)ₙ- wherein * is a binding site to the formula (S-7), and n is an integer of 1 to 5 and preferably 2 or 3.

In addition, another example which can be adopted as a substrate S includes a group represented by the following formula (S-8). 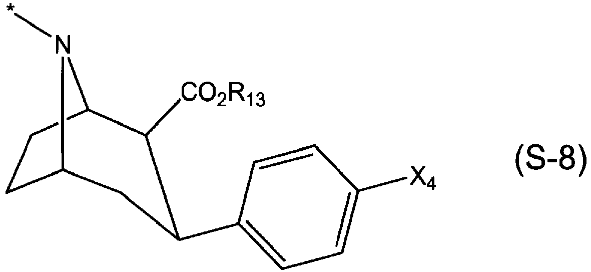 wherein X₄ is a halogen atom or a methyl group, R₁₃ is a hydrogen atom or an alkyl group having 1 to 6 carbon atoms, and the asterisk represents a binding site to L.

In the formula (S-8), X₄ is preferably a halogen atom and R₁₃ is preferably a methyl group. In addition, in a case where S is a group represented by the formula (S-7), L is preferably a group presented by *-(CH₂)ₙ- wherein * is a binding site to the formula (S-7), and n is an integer of 1 to 5 and preferably n is 3.

Examples of the radioactive labeled compound of the general formula (1) obtained from the labeling precursor compound of the present invention include various compounds which are used as a radiopharmaceutical, preferably a diagnostic drug by a positron emission tomography (PET) method. For example, in a case where the formula (S-3) is adopted as the substrate S, an amyloid affinity compound disclosed in WO 2007/135890 A may be mentioned. In addition, in a case where the formula (S-4) is adopted as the substrate S, a compound for imaging myocardial perfusion (for example, Flurpiridaz and the like) disclosed in WO 2005/079391 A may be mentioned by way of example. In addition, in a case where the formula (S-5) is adopted as the substrate S, a compound for imaging amyloid, such as Florbetapir and Florbetaben may be mentioned by way of example. In addition, in a case where the formula (S-6) is adopted as the substrate S, a compound for imaging a tumor, such as O-(2-fluoroethyl)-L-tyrosine (FET) may be mentioned by way of example. In addition, in a case where the formula (S-7) is adopted as the substrate S, a compound for image diagnosis of adrenal gland disease disclosed in WO 2015/199205 A may be mentioned by way of example. In addition, in a case where the formula (S-7) is adopted as the substrate S, a compound for mapping a monoamine reuptake site (for example, FP-CIT) disclosed in WO 99/01184 A may be mentioned by way of example.

The radioactive fluorine-labeling precursor compound of the present invention can be produced by, for example, acting a sulfonyl fluoride corresponding to a leaving group and diazabicycloundecene (DBU) on a compound (OH form) in which a hydroxyl group is bonded to a site where radioactive fluorine is to be introduced, as shown in SCHEME 1 below. Note that, in SCHEME 1 below, S, L, R¹ to R³, and p are the same as those mentioned above concerning the general formula (2), and X is a halogen atom.

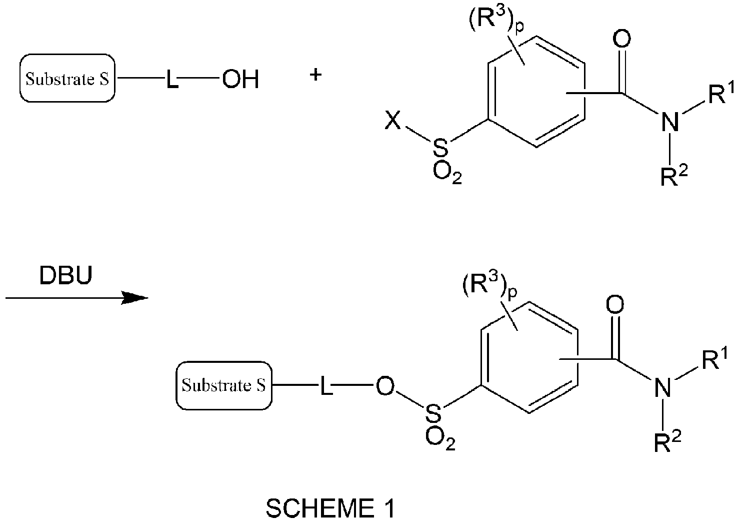

### 2. Production method of radioactive fluorine-labeled compound using radioactive fluorine-labeling precursor compound

According to the present invention, a radioactive fluorine-labeled compound represented by the general formula (1) can be produced by a step of allowing a radioactive fluorine-labeling precursor compound represented by the general formula (2) to react with [¹⁸F]fluoride ion (radioactive fluorine labeling reaction step) .

The radioactive fluorine labeling reaction is preferably performed in the presence of a base in an inert solvent. Specifically, the compound represented by the general formula (1) can be obtained by performing the reaction in an appropriate solvent such as an aprotic solvent, e.g., acetonitrile, N,N-dimethylformamide or dimethyl sulfoxide at a temperature of 20 to 120°C using a [¹⁸F]fluoride ion aqueous solution produced from [¹⁸O]water by cyclotron as the [¹⁸F]fluoride ion and using a base exemplified by tetrabutylammonium or potassium carbonate/Kryptofix 222. The radioactive fluorine labeling reaction can be performed with a synthesis apparatus equipped with a reaction vessel and a shield. In addition, the synthesis apparatus may be an automatic synthesis apparatus in which all steps are automated.

In the above reaction step, by-products such as an unreacted precursor compound (that is, a compound represented by the general formula (2)) and an OH form represented by the following general formula (3) coexist with the target compound represented by the general formula (1). 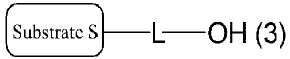 wherein S and L are the same as in the general formula (1).

The purification of the target compound represented by the general formula (1) can be performed in accordance with a solid phase extraction method using a reverse-phase cartridge column. Specifically, the unreacted precursor compound (that is, the compound represented by the general formula (2)) is usually higher in lipophilicity, in other words, higher in hydrophobicity than the target compound represented by the general formula (1). Accordingly, a method utilizing such a difference in hydrophobicity may be used, which may be exemplified by a method in which a reaction mixture obtained in the radioactive fluorine labeling reaction step is added to a reverse-phase cartridge column filled with octadecyl silica gel or the like, [¹⁸F]fluoride ion is separated, and then an appropriate elution solvent is allowed to pass through the above column, such that the compound of the general formula (1) which is the object compound can be eluted to be separated and collected. Examples of the elution solvent include water-soluble solvents such as acetonitrile, ethanol, t-butanol and methanol, or a mixed liquid of these with water. The compound of the general formula (1) which is the collected target compound can be subjected to deprotection and the like, if necessary, to be an object compound.

### Examples

Hereinafter, the present invention is described more specifically by way of examples; however, the present invention is not limited only to the following examples.

It is to be noted that in the following examples, the names of individual compounds used in experiments are defined as shown in Table 1.

**Table 1: Compound No. and Structural Formula**

| Compound | Structure |
|---|---|
| Precursor Compound 1 | 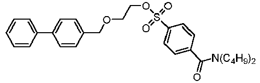 |
| Precursor Compound 2 | 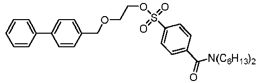 |
| Precursor Compound 3 | 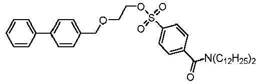 |
| Precursor Compound 4 | 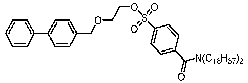 |
| Precursor Compound 5 | 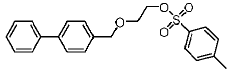 |
| Precursor Compound 6 | 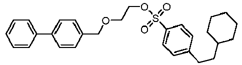 |
| Unlabeled Compound 1 | 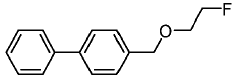 |

In the examples, the molecular structure of the individual compounds was identified based on nuclear magnetic resonance (NMR) spectra. AVANCE III HD (manufactured by BRUKER Japan K.K.) was used as an NMR apparatus and deuterated chloroform was used as a solvent. ¹H-NMR was measured at a resonance frequency of 500 MHz. ¹³C-NMR was measured at a resonance frequency of 125 MHz. All chemical shifts are given in terms of ppm on a delta scale (δ). Fine splittings of signals were indicated using abbreviations (s: singlet, d: doublet, t: triplet, dd: double doublet, dt: double triplet, dq: double quartet, m: multiplet, and br: broad).

Hereinafter, the term "room temperature" in the examples means 25°C.

In a synthesis example of each compound, each step in the compound synthesis was repeated plural times if necessary to secure an amount required for use as an intermediate or the like in other syntheses.

### Example 1: Synthesis of precursor compound 1

According to the following scheme, 2-([1,1'-biphenyl]-4-ylmethoxy)ethyl-4-(dibutylcarbamoyl)benzenesulfonate (precursor compound 1) was synthesized.

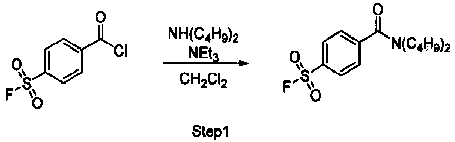

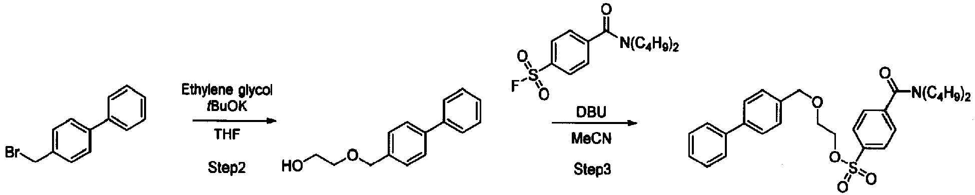

### Step 1: Synthesis of 4-(dibutylcarbamoyl)benzenesulfonic acid fluoride

Dibutylamine (280 µL, 1.60 mmol) was dissolved in dichloromethane (12 mL), triethylamine (0.25 mL, 1.8 mmol) was added to the resulting solution, the solution was cooled to 0°C, and then 4-fluorosulfonyl benzoic acid chloride (233 mg, 1.05 mmol) was added to the cooled solution and the solution was stirred at 0°C for 5 hours. After completion of the reaction, the reaction solution was added to 1 mol/L hydrochloric acid and extraction with ethyl acetate was performed two times. The combined ethyl acetate layer was washed with a saturated aqueous sodium hydrogen carbonate solution and a saturated saline solution, dried with anhydrous magnesium sulfate, and concentrated under reduced pressure, and then the obtained crude product was purified by silica gel column chromatography (eluent: hexane/ethyl acetate = 3 : 1) to obtain 4-(dibutylcarbamoyl)benzenesulfonic acid fluoride (191 mg, 0.61 mmol).

¹H-NMR: δ 8.06(d,2H,J=8.5Hz), 7.59(d,2H,J=8.0Hz), 3.51(t,2H,J=7.5Hz), 3.13(t,2H,J=7.5Hz), 1.69-1.63(m,2H), 1.58(m,2H), 1.52-1.46(m,2H), 1.43-1.38(m,2H), 1.19-1.11(m,2H), 0.99(t,3H,J=7.5Hz), 0.81(t,3H,J=7.5Hz)

### Step 2: Synthesis of 2-([1,1'-biphenyl]-4-ylmethoxy)ethanol

Potassium t-butoxide (444 mg, 4.4 mmol) was dissolved in ethylene glycol (4.4 mL, 78.9 mmol), a solution obtained by dissolving 4-(bromomethyl)-1,1'-biphenyl (1.8 g, 4.386 mmol) in tetrahydrofuran (20 mL) was added to the resulting solution, and the solution was stirred at 65°C for 6.5 hours. Potassium t-butoxide (101 mg, 1.1 mmol) was added and stirred at 65°C for 1.5 hours. After completion of the reaction, the reaction solution was added to 0.1 mol/L hydrochloric acid and extraction with ethyl acetate was performed two times. The combined ethyl acetate layer was washed with water, dried with anhydrous magnesium sulfate, and concentrated under reduced pressure, and then the obtained crude product was purified by silica gel column chromatography (eluent: hexane/ethyl acetate = 3 : 1) to obtain 2-([1,1'-biphenyl]-4-ylmethoxy)ethanol (967 mg, 4.24 mmol).

¹H-NMR: δ 7.60-7.58(m,4H), 7.46-7.41(m,4H), 7.37-7.34(m,1H), 4.61(s,2H), 3.79(dd,2H,J=6.0,4.0Hz), 3.64(dd,2H,J=6.0,4.0Hz)

### Step 3: Synthesis of precursor compound 1

2-([1,1'-biphenyl]-4-ylmethoxy)ethanol (52 mg, 0.22 mmol) was dissolved in acetonitrile (2.0 mL), the solution was cooled to 0°C, and then diazabicycloundecene (79 µL, 0.50 mmol) and 4-(dibutylcarbamoyl)benzenesulfonic acid fluoride (88 mg, 0.26 mmol) were added to the cooled solution and the solution was stirred at room temperature for 1 hour. After completion of the reaction, water was added to the reaction solution and extraction with ethyl acetate was performed two times. The combined ethyl acetate layer was washed with water and a saturated saline solution, dried with anhydrous magnesium sulfate, and concentrated under reduced pressure, and then the obtained crude product was purified by silica gel column chromatography (eluent: hexane/ethyl acetate = 75/25) to obtain the precursor compound 1 (70 mg, 0.14 mmol).

¹H-NMR: δ 7.95(d,2H,J=8.5Hz), 7.60-7.56(m,4H), 7.49(d,2H,J=8.5Hz), 7.46-7.43(m,2H), 7.37-7.35(m,3H), 4.54(s,2H), 4.25(t,2H,J=4.5Hz), 3.70(t,2H,J=4.5Hz), 3.49(t,2H,J=7.5Hz), 3.11(t,2H,J=7.5Hz), 1.68-1.60(m,2H), 1.49-1.36(m,4H), 1.18-1.08(m,2H) 0.98(t,3H,J=7.3Hz), 0.78(t,3H,J=7.3Hz)

### Example 2: Synthesis of precursor compound 2

According to the following scheme, 2-([1,1'-biphenyl]-4-ylmethoxy)ethyl-4-(dihexylcarbamoyl)benzenesulfonate (precursor compound 2) was synthesized.

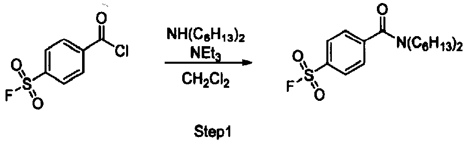

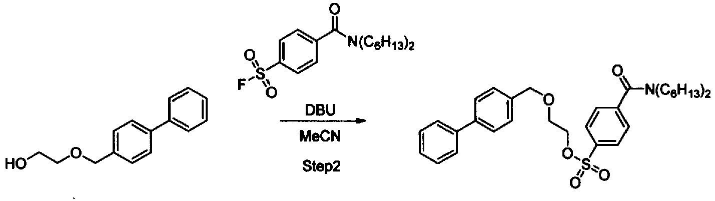

### Step 1: Synthesis of 4-(dihexylcarbamoyl)benzenesulfonic acid fluoride

Dihexylamine (176 µL, 0.75 mol) was dissolved in dichloromethane (12 mL), triethylamine (0.13 mL, 1.25 mmol) was added to the resulting solution, the solution was cooled to 0°C, and then 4-fluorosulfonyl benzoic acid chloride (151 mg, 0.68 mmol) was added to the cooled solution and the solution was stirred at 0°C for 3 hours. After completion of the reaction, the reaction solution was added to 1 mol/L hydrochloric acid and extraction with ethyl acetate was performed two times. The combined ethyl acetate layer was washed with a saturated aqueous sodium hydrogen carbonate solution and a saturated saline solution, dried with anhydrous magnesium sulfate, and concentrated under reduced pressure, and then the obtained crude product was purified by silica gel column chromatography (eluent: hexane/ethyl acetate = 3 : 1) to obtain 4-(dihexylcarbamoyl)benzenesulfonic acid fluoride (181 mg, 0.49 mmol).

δ 8.06(d,2H,J=8.5Hz), 7.59(d,2H,J=8.0Hz), 3.49(t,2H,J=7.5Hz), 3.12(t,2H,J=7.5Hz), 1.70-1.64(m,2H), 1.52-1.48(m,2H), 1.40-1.32(m,6H) 1.25-1.18(m,2H), 1.18-1.08(m,4H), 0.93-0.90(m,3H), 0.84(t,3H,J=7.3z)

### Step 2: Synthesis of precursor compound 2

2-([1,1'-biphenyl]-4-ylmethoxy)ethanol (50 mg, 0.22 mmol) was dissolved in acetonitrile (2.0 mL), the solution was cooled to 0°C, and then diazabicycloundecene (79 µL, 0.50 mmol) and 4-(dibutylcarbamoyl)benzenesulfonic acid fluoride (85 mg, 0.22 mmol) were added to the cooled solution and the solution was stirred at room temperature for 1 hour. After completion of the reaction, water was added to the reaction solution and extraction with ethyl acetate was performed two times. The combined ethyl acetate layer was washed with water and a saturated saline solution, dried with anhydrous magnesium sulfate, and concentrated under reduced pressure, and then the obtained crude product was purified by silica gel column chromatography (eluent: hexane/ethyl acetate = 3 : 1) to obtain the precursor compound 2 (83 mg, 0.14 mmol).

¹H-NMR: δ 7.96(d,2H,J=8.5Hz), 7.60-7.57(m,4H), 7.48(d,2H,J=8.5Hz), 7.46-7.42(m,2H), 7.37-7.33(m,3H), 4.54(s,2H), 4.25(t,2H,J=4.8Hz), 3.70(t,2H,J=4.8Hz), 3.47(t,2H,J=7.8Hz), 3.10(t,2H,J=7.5Hz), 1.68-1.60(m,2H), 1.49-1.30(m,6H), 1.25-1.17(m,2H), 1.17-1.08(m,4H) 0.93-0.90(m,3H), 0.83(t,3H,J=7.3z)

### Example 3: Synthesis of precursor compound 3

According to the following scheme, 2-([1,1'-biphenyl]-4-ylmethoxy)ethyl-4-(didodecylcarbamoyl)benzenesulfonate (precursor compound 3) was synthesized.

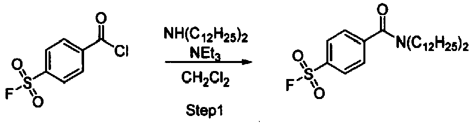

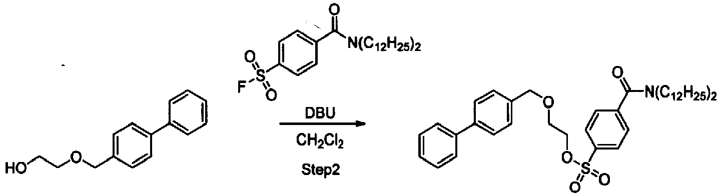

### Step 1: Synthesis of 4-(didodecylcarbamoyl)benzenesulfonic acid fluoride

Didodecylamine (500 mg, 2.70 mmol) was dissolved in dichloromethane (23 mL), triethylamine (0.63 mL, 4.50 mmol) was added to the resulting solution, the solution was cooled to 0°C, and then 4-fluorosulfonyl benzoic acid chloride (500 mg, 2.25 mmol) was added to the cooled solution and the solution was stirred at room temperature for 18 hours. After completion of the reaction, 1 mol/L hydrochloric acid was added to the reaction solution and extraction with ethyl acetate was performed three times. The combined ethyl acetate layer was washed with a saturated aqueous sodium hydrogen carbonate solution and a saturated saline solution, dried with anhydrous sodium sulfate, and concentrated under reduced pressure, and then the obtained crude product was purified by silica gel column chromatography (eluent: toluene/ethyl acetate = 40 : 1) to obtain 4-(didodecylcarbamoyl)benzenesulfonic acid fluoride (627 mg, 1.16 mmol).

¹H-NMR:δ 8.05(d,2H,J=8.3Hz), 7.59(d,2H,J=8.3Hz), 3.49(t,2H,J=7.6Hz), 3.11(t,2H,J=7.4Hz), 1.65(br,2H), 1.49(br,2H), 1.35-1.09(m,36H), 0.88(t,6H,J=6.7Hz)

### Step 2: Synthesis of 2-([1,1'-biphenyl]-4-ylmethoxy)ethyl-4-(didodecylcarbamoyl)benzenesulfonate (precursor compound 3)

2-([1,1'-biphenyl]-4-ylmethoxy)ethanol (32 mg, 0.14 mmol) was dissolved in dichloromethane (1.4 mL), the solution was cooled at 0°C, and then diazabicycloundecene (50 µL, 0.33 mmol) and 4-(didodecylcarbamoyl)benzenesulfonic acid fluoride (90 mg, 0.17 mmol) were added to the cooled solution and the solution was stirred at room temperature for 18 hours. After completion of the reaction, water was added to the reaction solution and extraction with chloroform was performed three times. The combined ethyl acetate layer was dried with anhydrous sodium sulfate, and concentrated under reduced pressure, and then the obtained crude product was purified by silica gel column chromatography (eluent: chloroform) to obtain 2-([1,1'-biphenyl]-4-ylmethoxy)ethyl-4-(didodecylcarbamoyl)benzenesulfonate (54 mg, 0.07 mmol).

¹H-NMR:δ 7.96-7.94(m,2H), 7.60-7.57(m,4H), 7.49-7.42(m,4H), 7.37-7.35(m,3H), 4.54(s,2H), 4.25(t,2H,J=4.6Hz), 3.70(t,2H,J=4.6Hz), 3.47(t,2H,J=7.6Hz), 3.09(t,2H,J=7.2Hz), 1.63(br,2H),1.46(br,2H), 1.35-1.07(m,36H), 0.88-0.87(m,6H)

### Example 4: Synthesis of precursor compound 4

According to the following scheme, 2-([1,1'-biphenyl]-4-ylmethoxy)ethyl-4-(dioctadecylcarbamoyl)benzenesulfonate (precursor compound 4) was synthesized.

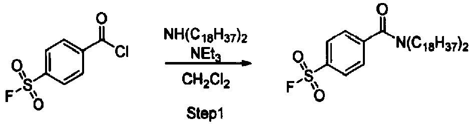

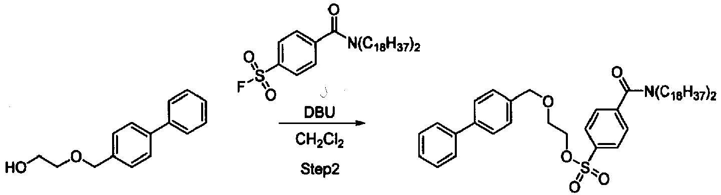

### Step 1: Synthesis of 4-(dioctadecylcarbamoyl)benzenesulfonic acid fluoride

Dioctadecylamine (282 mg, 0.54 mmol) was dissolved in dichloromethane (1 mL), triethylamine (0.13 mL, 0.90 mmol) was added to the resulting solution, the solution was cooled to 0°C, and then 4-fluorosulfonyl benzoic acid chloride (100 mg, 0.45 mmol) was added to the cooled solution and the solution was stirred at room temperature for 18 hours. After completion of the reaction, water was added to the reaction solution and extraction with chloroform was performed three times. The combined chloroform layer was dried with anhydrous sodium sulfate, and concentrated under reduced pressure, and then the obtained crude product was purified by silica gel column chromatography (eluent: hexane/ethyl acetate = 5/1) to obtain 4-(dioctadecylcarbamoyl)benzenesulfonic acid fluoride (208 mg, 0.29 mmol).

¹H-NMR:δ 8.05(d,2H,J=8.4Hz), 7.60(d,2H,J=8.4Hz), 3.49(t,2H,J=7.6Hz), 3.11(t,2H,J=7.6Hz), 1.66(br,2H), 1.49(br,2H), 1.36-1.10(m,60H), 0.88(t,6H,J=7.0)

### Step 2: Synthesis of 2-([1,1'-biphenyl]-4-ylmethoxy)ethyl-4-(dioctadecylcarbamoyl)benzenesulfonate (precursor compound 4)

2-([1,1'-biphenyl]-4-ylmethoxy)ethan-1-ol (30 mg, 0.13 mmol) was dissolved in dichloromethane (1.3 mL), the solution was cooled to 0°C, and then diazabicycloundecene (47 µL, 0.32 mmol) and 4-(dioctadecylcarbamoyl)benzenesulfonic acid fluoride (112 mg, 0.16 mmol) were added to the cooled solution and the solution was stirred at room temperature for 18 hours. After completion of the reaction, water was added to the reaction solution and extraction with chloroform was performed three times. The combined chloroform layer was dried with anhydrous sodium sulfate, and concentrated under reduced pressure, and then the obtained crude product was purified by silica gel column chromatography (eluent: chloroform) to obtain 2-([1,1'-biphenyl]-4-ylmethoxy)ethyl-4-(dioctadecylcarbamoyl)benzenesulfonate (63 mg, 0.07 mmol).

¹H-NMR:δ 7.96-7.94(m,2H), 7.60-7.57(m,4H), 7.49-7.42(m,4H), 7.37-7.35(m,3H), 4.54(s,2H), 4.25(t,2H,J=4.7Hz), 3.70(t,2H,J=4.7Hz), 3.50-3.45(m,2H), 3.10-3.08(m,2H), 1.64-1.62(m,2H), 1.47-1.46(m,2H), 1.34-1.04(m,60H), 0.88(t,6H,J=6.75Hz)

### Comparative Example 1: Synthesis of 2-([1,1'-biphenyl]-4-ylmethoxy)ethyl-4-methylbenzenesulfonate (precursor compound 5)

According to the following scheme, the precursor compound 5 was synthesized.

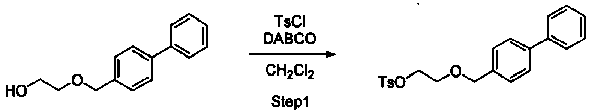

### Step 1: Synthesis of 2-([1,1'-biphenyl]-4-ylmethoxy)ethyl-4-methylbenzenesulfonate

2-([1,1'-biphenyl]-4-ylmethoxy)ethanol (55 mg, 0.24 mmol) was dissolved in dichloromethane (2 mL), and 1,4-diazabicyclo[2,2,2]octane (86 mg, 0.77 mmol) and p-toluenesulfonyl chloride (69 mg, 0.36 mmol) were added to the resulting solution and the solution was stirred at room temperature for 4.5 hours. After completion of the reaction, water was added to the reaction solution and extraction with ethyl acetate was performed two times. The combined ethyl acetate layer was dried with anhydrous magnesium sulfate, and concentrated under reduced pressure, and then the obtained crude product was purified by silica gel column chromatography (eluent: hexane/ethyl acetate = 3 : 1) to obtain 4-methylbenzenesulfonic acid 2-([1,1'-biphenyl]-4-ylmethoxy)ethyl (64 mg, 0.17 mmol).

¹H-NMR:δ 7.81(d,2H,J=8.0Hz), 7.60-7.54(m,4H), 7.47-7.43(m,2H), 7.37-7.30(m,5H), 4.53(s,2H), 4.22(t,2H,J=4.8Hz), 3.70(t,2H,J=4.8Hz), 2.43(s,3H)

### Comparative Example 2: Synthesis of precursor compound 6

According to the following scheme, 4-(2-cyclohexylethyl)benzenesulfonic acid 2-([1,1'-biphenyl]-4-ylmethoxy)ethyl (precursor compound 6) was synthesized.

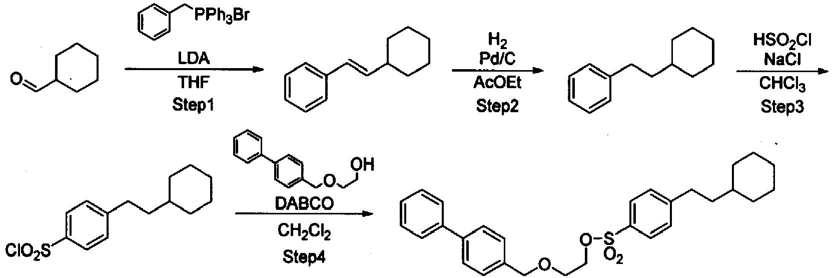

### Step 4: Synthesis of precursor compound 6

2-([1,1'-diphenyl]-4-ylmethoxy)-ethanol (50.6 mg, 0.222 mmol) was dissolved in dichloromethane (2.0 mL) and cooled to 0°C, and then the resultant solution was supplemented with a solution in which 1,4-diazabicyclo[2.2.2]octane (37.4 mg, 0.333 mmol) was dissolved in dichloromethane (2.0 mL) together with 4-(2-cyclohexyl-ethyl)-benzenesulfonyl chloride (76.4 mg, 0.266 mmol) obtained by performing the Steps 1 to 3 in accordance with the method described in Example 1 of WO 2011/006610 A. After stirring for 30 minutes, the reaction was stopped by addition of a saturated aqueous sodium hydrogen carbonate solution and extraction with dichloromethane was performed three times. The combined dichloromethane layer was washed with water and a saturated saline solution, dried over sodium sulfate, and concentrated under reduced pressure. The obtained crude product was purified by silica gel column chromatography (eluent: hexane/ethyl acetate = 4 : 1) to obtain 4-(2-cyclohexylethyl)benzenesulfonic acid 2-([1,1'-biphenyl]-4-ylmethoxy)ethyl (75.6 mg, 0.158 mmol).

¹H-NMR:δ 7.81(d,2H,J=8.4Hz), 7.59-7.55(m,4H), 7.44(t,2H,J=7.6Hz), 7.37-7.30(m,5H), 4.53(s,2H), 4.23(t,2H,J=4.8Hz), 3.70(t,2H,J=4.8Hz), 2.67(t,2H,J=8.2Hz), 1.74-1.64(m,5H), 1.51-1.47(m,2H), 1.25-1.13(m,4H), 0.96-0.88(m,2H)

### Reference Example 1: Synthesis of unlabeled compound 1

According to the following scheme, 4-([2-fluoroethoxy]methyl)-1,1'-biphenyl (unlabeled compound 1) was synthesized.

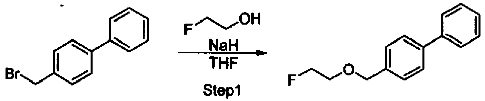

### Step 1: Synthesis of 4-([2-fluoroethoxy]methyl)-1,1'-biphenyl

2-Fluoroethanol (86 mg, 0.77 mmol) was dissolved in tetrahydrofuran (1.4 mL) and cooled to 0°C, and sodium hydride (3.2 mg, 0.14 mmoL) was added to the cooled solution and the solution was stirred for 10 minutes. Subsequently, 4-(bromomethyl)-1,1'-biphenyl (50 mg, 0.20 mmol) was added and stirred at room temperature for 18 hours. After completion of the reaction, a saturated aqueous ammonium chloride solution was added to the reaction solution and extraction with chloroform was performed three times. The combined chloroform layer was washed with water, dried with anhydrous sodium sulfate, concentrated under reduced pressure, and then the obtained crude product was purified by silica gel column chromatography (eluent: hexane/ethyl acetate = 10/1) to obtain 4-([2-fluoroethoxy]methyl)-1,1'-biphenyl (5.8 mg, 0.03 mmol).

¹H-NMR:δ 7.60-7.58(m,4H), 7.45-7.42(m,4H), 7.36-7.33(m,1H), 4.65(s,2H), 4.61(dt,2H,J=47.7Hz,4.2Hz), 3.76(dt,2H,J=29.4Hz,4.2Hz)

### Example 5: Preparation of radioactive fluorinated 4-[(2-[¹⁸F]fluoroethoxy)methyl]-1,1'-biphenyl using precursor compounds 1 to 4

An aqueous potassium carbonate solution (50 µmol/L, 0.2 mL) and a solution of Kryptofix 222 (trade name, manufactured by Merck KGaA) (12 mg, 37.2 µmol) dissolved in acetonitrile (0.6 mL) were added to [¹⁸F]fluoride ioncontaining [¹⁸O]water. The resulting solution was heated at 110°C in a flow of argon gas to evaporate water, and then supplemented with acetonitrile (0.5 mL × 3) and azeotropically evaporated to dryness. A solution of each of the precursor compounds 1 to 6 (8 µmol) synthesized in accordance with the methods shown in Examples 1 to 6 dissolved in acetonitrile (0.5 mL) was added to the dried residue, and the mixture was heated at 90°C for 5 minutes. After completion of the reaction, the mixture was analyzed by a thin layer chromatography (TLC) under the following conditions, and then water for injection (10 mL) was added to the mixture. Then, the mixture was allowed to pass through Sep-Pak (registered trademark) C18 Plas (trade name, manufactured by Nippon Waters K.K.), such that 4-[(2-[¹⁸F]fluoroethoxy)methyl]-1,1'-biphenyl was absorbed and collected onto the corresponding column. After the column was washed with water (10 mL), a mixed liquid (4 mL) of water/acetonitrile = 1:3 was allowed to pass through the column to elute 4-[(2-[¹⁸F]fluoroethoxy)methyl]-1,1'-biphenyl.

The 4-[(2-[¹⁸F]fluoroethoxy)methyl]-1,1'-biphenyl obtained by the operation described above was subjected to an HPLC analysis under the following conditions. Note that the identification was performed by confirming whether or not its Rf value on the TLC plate is the same as that of the unlabeled compound of 4-[(2-fluoroethoxy)methyl]-1,1'-biphenyl synthesized with Reference Example 1.

### TLC condition

Plate: TLC glass plate Silica gel 60F₂₅₄
Developing solvent: hexane/ethyl acetate = 3 : 1

### HPLC condition

Column: CAPCELLPAKC18MGII (trade name, manufactured by Shiseido Company, Limited, particle size: 5 µm, size: 4.6 mmϕ × 150 mm)
Mobile phase: 20 mmol/L ammonium acetate buffer solution (pH = 6.0)/acetonitrile = 70/30 → 30/70 (0 → 30 minutes), 30/70 (30 → 45 minutes), 30/70 → 1/99 (45 → 46 minutes), 1/99 (46 → 100 minutes)
Flow rate: 1.0 mL/min
Detector: ultraviolet-visible absorption photometer (detection wavelength: 254 nm)

### Comparative Example 3: Preparation of radioactive fluorinated 4-[(2- [¹⁸F]fluoroethoxy)methyl]-1,1'-biphenyl using conventional precursor compound

The preparation was performed in the same manner as in Example 5 except that, as a precursor compound, the precursor compound 5 or 6 synthesized in accordance with the method shown in Comparative Example 1 or 2 was used.

### Evaluation 1: Evaluation of labeling reaction for fluorine-labeled compound

Table 2 shows the amounts of radioactivity used in Example 5 and Comparative Example 3, and the amount of radioactivity and [¹⁸F] fluorination rate of the obtained product (4-[(2-[¹⁸F]fluoroethoxy)methyl]-1,1'-biphenyl). A peak area ratio of 4-[(2-[¹⁸F]fluoroethoxy)methyl]-1,1'-biphenyl subjected to the TLC analysis after completion of the reaction was taken as a [¹⁸F] fluorination rate.

As shown in Table 2, by using the precursor compounds 1 to 4 of the Examples, the approximately same [¹⁸F] fluorination rate as those of the conventional precursor compounds 5 and 6 was obtained.

**Table 2**

| | Radioactivity of [¹⁸F]F⁻ water used (Value corrected at start of synthesis) | Amount of radioactivity of product (Measurement time) | [¹⁸F] fluorination rate at the time of completing reaction |
|---|---|---|---|
| Precursor Compound 1 | 443 MBq | 241 MBq (44 minutes after start of synthesis) | 75% |
| Precursor Compound 2 | 234 MBq | 177 MBq (44 minutes after start of synthesis) | 82% |
| Precursor Compound 3 | 488 MBq | 250 MBq (35 minutes after start of synthesis) | 67% |
| Precursor Compound 4 | 676 MBq | 276 MBq (41 minutes after start of synthesis) | 65% |
| Precursor Compound 5 | 158 MBq | 78.4 MBq (65 minutes after start of synthesis) | 78% |
| Precursor Compound 6 | 507 MBq | 256 MBq (60 minutes after start of synthesis) | 66% |

### Evaluation 2: Evaluation of impurities

Table 3 shows the evaluation results obtained by an HPLC analysis of the amount of nonradioactive impurities in the 4-[(2-[¹⁸F]fluoroethoxy)methyl]-1,1'-biphenyl obtained in Example 5 and Comparative Example 3. A mixed amount of the precursor compound was quantitatively determined with a calibration curve prepared using a standard sample. In addition, a collection rate was shown as a collection rate with respect to the amount of precursor compound used in the radioactive fluorination reaction. The amount of impurities having unknown structures was converted to the amount of OH form (2-([1,1'-biphenyl]-4-ylmethoxy)ethan-1-ol) for evaluation.

As a result, as shown in Table 3, all of the precursor compounds 1 to 4 of the Examples showed smaller amounts of precursor contamination than the conventional precursor compounds 5 and 6. In addition, the precursor compounds 1, 3, and 4 were also smaller in amounts of nonradioactive impurities having unknown structures than the conventional compound.

**Table 3**

| | Mixed amount of precursor compound (Collection rate) | Mixed amount of nonradioactive impurities* having unknown structures (Collection rate) |
|---|---|---|
| Precursor Compound 1 | 11 µg/mL (1%) | 335 µg/mL (32%) |
| Precursor Compound 2 | 12 µg/mL (1%) | 401 µg/mL (34%) |
| Precursor Compound 3 | Lower than detection limit * (0%) | 310 µg/mL (21%) |
| Precursor Compound 4 | Lower than detection limit * (0%) | 290 µg/mL (26%) |
| Precursor Compound 5 | 303µg/mL (40%) | 354 µg/mL (46%) |
| Precursor Compound 6 | 60µg/mL (6%) | 191 µg/mL (20%) |

| | | |
|---|---|---|
| *Detection limit: 1 µg/mL | | |

### Example 6: Synthesis of precursor compound 7

According to the following scheme, 2-(4-(6-imidazo[1,2-a]pyridine-2-yl)phenoxy)ethyl-4-(didodecylcarbamoyl)benzenesulfonate (precursor compound 7) was synthesized.

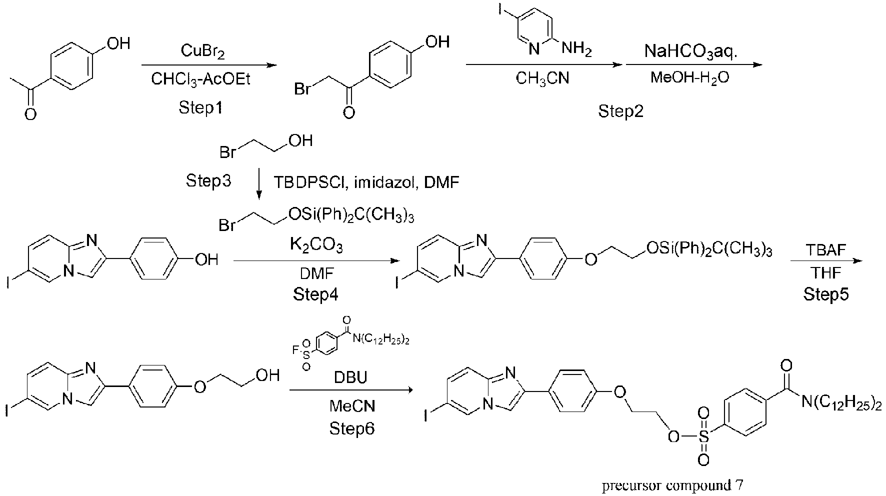

### Step 6: Synthesis of precursor compound 7

2-[4'-(2-hydroxyethoxy)phenyl]-6-iodoimidazo[1,2-a]pyridine (100 mg, 0.263 mmol) obtained by performing the Steps 1 to 5 in accordance with the method described in Example 11-14 of WO 2007/135890 A was dissolved in acetonitrile (10.0 mL) and cooled to 0°C, and then the resultant solution was supplemented with a solution in which 1,8-diazabicyclo[5.4.0]undecene (78.6 µL, 0.526 mmol) was dissolved in acetonitrile (2.0 mL) together with 4-didodecylcarbamoyl benzene sulfonic acid fluoride (185 mg, 0.342 mmol) obtained by performing the Step 1 in accordance with the method described in Example 3. After warming to room temperature and stirring for 3 hours, the reaction was stopped by addition of water and extraction with ethyl acetate was performed three times. The combined ethyl acetate layer was washed with a saturated aqueous sodium hydrogen carbonate solution, water, and a saturated saline solution, dried over sodium sulfate, and concentrated under reduced pressure. The obtained crude product was purified by silica gel column chromatography (eluent: chloroform) to obtain 2-(4-(6-imidazo[1,2-a]pyridine-2-yl)phenoxy)ethyl-4-(didodecylcarbamoyl)benzenesulfonate (198 mg, 0.220 mmol) (precursor compound 7).

¹H-NMR:δ 8.37(s,1H), 7.98(d,2H,J=8.4Hz), 7.84(d,2H,J=8.8Hz), 7.7(s,1H), 7.52(d,2H,J=8.4Hz), 7.40(d,1H,J=9.4Hz), 7.32(d,1H,J=9.4Hz), 6.88(d,2H,J=8.8Hz), 4.42(t,2H,J=4.6Hz), 3.48(t,2H,J=4.6Hz), 3.48(t,2H,J=7.6Hz), 3.11(t,2H,J=7.6Hz), 1.65-1.45(m,12H), 1.36-1.08(m,29H), 0.89-0.86(m,5H)

### Example 7: Synthesis of amyloid beta imaging agent

An amyloid beta imaging agent, 2-[4'-(2"-[¹⁸F]fluoroethoxy)phenyl]-6-iodoimidazo[1,2-a]pyridine (¹⁸F labeled compound 1-9 in WO 2007/135890 A) was produced by using the precursor compound 7 synthesized in accordance with the method shown in Example 6.

An aqueous potassium carbonate solution (66 µmol/L, 0.3 mL) and a solution of Kryptofix 222 (trade name, manufactured by Merck KGaA) (15 mg, 39.9 µmol) dissolved in acetonitrile (1.5 mL) were added to [¹⁸F]fluoride ioncontaining [¹⁸O]water (the amount of radioactivity: 533 MBq, the value corrected at the start of synthesis). The resulting solution was heated at 110°C in a flow of argon gas to evaporate water, and then supplemented with acetonitrile (0.5 mL × 3) and azeotropically evaporated to dryness. A solution of the precursor compound 7 (30 mg, 33 µmol) synthesized with the example described above dissolved in dimethyl sulfoxide (0.5 mL) was added to the mixture and heated at 110°C for 10 minutes. After completion of the reaction, water for injection (10 mL) was added to the mixture, and the mixture was allowed to pass through Sep-Pak (registered trademark) C18 Plas (trade name, manufactured by Nippon Waters K.K.). Then, the column was washed with water (10 mL) and a mixed liquid (2 mL) of water/acetonitrile = 1:1, and then 2-[4'-(2"-[¹⁸F]fluoroethoxy)phenyl]-6-iodoimidazo[1,2-a]pyridine was eluted with a mixed liquid (3 mL) of water/acetonitrile = 1 : 3. The obtained amount of radioactivity was 186 MBq (58 minutes after the start of synthesis). In addition, as a result of the HPLC analysis under the following conditions, it was confirmed that 4 µg/mL of the unreacted precursor compound 7 was mixed. In case where a toluene sulfonic acid ester group is used as a leaving group, instead of the sulfonic acid ester group having an alkyl amide tag introduced according to the present invention, a mixed amount of the unreacted precursor compound was 650 µg/mL. Thus, it was confirmed that an amyloid beta imaging agent low in contamination of the unreacted precursor compound was able to be synthesized by the present invention even without the HPLC purification.

### HPLC condition

Column: YMC-Pack Pro C8 (trade name, manufactured by YMC CO., LTD., particle size: 5 µm, size: 4.6 mmϕ × 150 mm)
Mobile phase: 10 mmol/L ammonium formate buffer solution (pH3)/acetonitrile = 100/0 → 70/30 (0 → 20 minutes), 70/3 → 10/90 (20 → 30 minutes), 10/90 (30 → 70 minutes)
Flow rate: 1.0 mL/min
Detector: ultraviolet-visible absorption photometer (detection wavelength: 260 nm)

### Example 8: Synthesis of precursor compound 8

According to the following scheme, 2-(2-{5-[(1H-imidazole-1-yl)methyl]pyridine-3-yl}-6-chloro-5-fluoro-1H-benzimidazole-1-yl)ethyl-4-(didodecylcarbamoyl)benzenesulfonate (precursor compound 8) was synthesized.

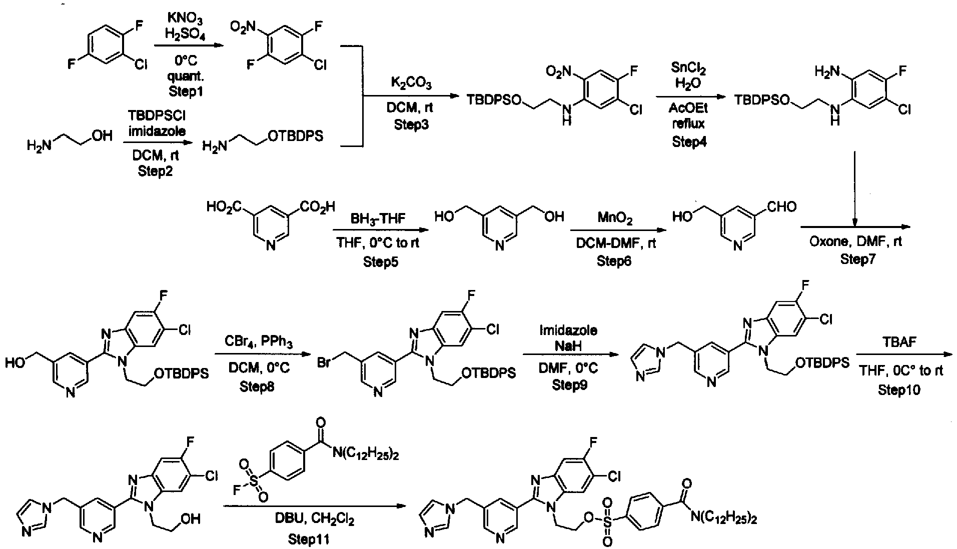

### Step 1: Synthesis of precursor compound 8

2-{6-chloro-5-fluoro-2-[5-(imidazole-1-ylmethyl)pyridine-3-yl]benzimidazole-1-yl}ethanol (57.3 mg, 0.154 mmol) obtained by performing the Steps 1 to 10 in accordance with the method described in Example 2 of WO 2015/199205 A was dissolved in dichloromethane (0.54 mL) and cooled to 0°C, and then the resultant solution was supplemented with a solution in which 1,8-diazabicyclo[5.4.0]undecene (55.3 µL, 0.370 mmol) was dissolved in dichloromethane (1.0 mL) together with 4-didodecylcarbamoyl benzene sulfonic acid fluoride (100 mg, 0.185 mmol) obtained by performing the Step 1 in accordance with the method described in Example 3. After warming to room temperature and stirring for 1 hour, the reaction was stopped by addition of water and extraction with dichloromethane was performed three times. The combined dichloromethane layer was washed with water and a saturated saline solution, dried over sodium sulfate, and concentrated under reduced pressure. The obtained crude product was purified by silica gel column chromatography (eluent: dichloromethane) to obtain 2-(2-{5-[(1H-imidazole-1-yl)methyl]pyridine-3-yl}-6-chloro-5-fluoro-1H-benzimidazole-1-yl)ethyl-4-(didodecylcarbamoyl)benzenesulfonate (precursor compound 8) (94 mg, 0.105 mmol).

¹H-NMR:δ 8.85(d,1H,J=2.1Hz), 8.63(d,1H,J=2.1Hz), 7.84(dd,1H,J=2.1,2.1Hz), 7.66(d,2H,J=8.4Hz), 7.66(s,1H),7.59(d,1H,J=9.0Hz), 7.43(d,2H,J=8.4Hz), 7.40(d,1H,J=6.1Hz), 7.14(t,1H,J=1.2Hz), 7.00(t,1H,J=1.2Hz), 5.29(s,2H), 4.47(t,2H,5.2Hz) 4.25(t,2H,J=5.2Hz), 3.47(t,2H,J=7.5Hz), 3.07(t,2H,J=7.5Hz), 1.65(br,2H), 1.47-1.43(m,2H), 1.36-1.06(m,36H), 0.88-0.86(m,6H)

### Example 9: Synthesis of aldosterone synthase imaging agent

An aldosterone synthase imaging agent, 6-chloro-5-fluoro-1-(2-[¹⁸F]fluoroethyl)-2-[5-(imidazole-1-ylmethyl)pyridine-3-yl]benzimidazole (¹⁸F labeled compound 100 in WO 2015/199205 A) was produced by using the precursor compound 8 synthesized with the method shown in Example 8.

An aqueous potassium carbonate solution (50 µmol/L, 0.25 mL) and a solution of Kryptofix 222 (trade name, manufactured by Merck KGaA) (14 mg, 37.2 µmol) dissolved in acetonitrile (0.7 mL) were added to [¹⁸F]fluoride ioncontaining [¹⁸O]water (the amount of radioactivity: 533 MBq, the value corrected at the start of synthesis). The resulting solution was heated at 110°C in a flow of argon gas to evaporate water, and then supplemented with acetonitrile (0.5 mL × 3) and azeotropically evaporated to dryness. A solution of the precursor compound 7 (8.5 mg, 9.5 µmol) synthesized with the example described above dissolved in dimethyl sulfoxide (0.5 mL) was added to the mixture and heated at 110°C for 10 minutes. After completion of the reaction, water for injection (10 mL) was added to the mixture, and the mixture was allowed to pass through Sep-Pak (registered trademark) C18 Plas (trade name, manufactured by Nippon Waters K.K.). Then, the column was washed with water (10 mL), and then 6-chloro-5-fluoro-1-(2-[¹⁸F]fluoroethyl)-2-[5-(imidazole-1-ylmethyl)pyridine-3-yl]benzimidazole was eluted with a mixed liquid (5 mL) of water/acetonitrile = 1:1. The obtained amount of radioactivity was 141 MBq (44 minutes after the start of synthesis). In addition, as a result of the HPLC analysis under the following conditions, it was confirmed that the unreacted precursor compound 8 was able to be removed to less than a detection limit value. In case where a toluene sulfonic acid ester group is used as a leaving group, instead of the sulfonic acid ester group having an alkyl amide tag introduced according to the present invention, a mixed amount of the unreacted precursor compound was 115 µg/mL. Thus, it was confirmed that an aldosterone synthase imaging agent low in contamination of the unreacted precursor compound was able to be synthesized by the present invention even without the HPLC purification.

### HPLC condition

Column: XBridge Phenyl (trade name, manufactured by Nippon Waters K.K., particle size: 3.5 um, size: 4.6 mmϕ × 100 mm)
Mobile phase: 10 mmol/L ammonium carbonate solution/methanol = 50/50 → 35/65 (0 → 10 minutes), 35/65 → 0/100 (10 → 25 minutes), 0/100 (25 → 50 minutes)
Flow rate: 1.0 mL/min
Detector: ultraviolet-visible absorption photometer (detection wavelength: 254 nm)

This application claims priority based on Japanese Patent Application No. 2017-042783 filed on March 7, 2017.

## Claims

1. A labeling precursor compound for a radioactive fluorine-labeled compound represented by the following general formula (1): 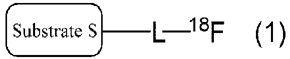 wherein S represents a substrate, and
L represents a straight alkyl group having 1 to 6 carbon atoms, which may contain an ether group,
the labeling precursor compound being represented by the following general formula (2): 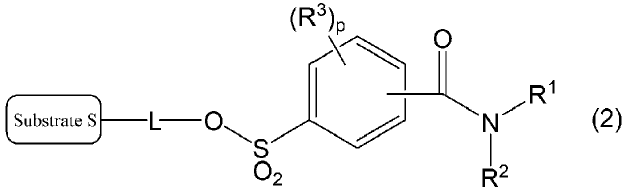
wherein S and L are the same as in the general formula (1),
R¹ and R² each independently represent a straight or branched alkyl group having 1 to 30 carbon atoms, or a substituted or unsubstituted monocyclic or condensed polycyclic aryl group,
R³ each independently represent an alkyl group having 1 to 4 carbon atoms or an alkoxy group having 1 to 4 carbon atoms, and
p represents an integer of 0 to 4,
**characterized in that** the radioactive fluorine-labeled compound represented by the general formula (1) has a clogP of -1.4 to 5.0, and a difference in clogP between the radioactive fluorine-labeled compound represented by the general formula (1) and the precursor compound represented by the general formula (2) is 2 or more.

2. The labeling precursor compound according to claim 1, wherein, in the general formulas (1) and (2), S is a group represented by the following formula (S-1) or (S-2): 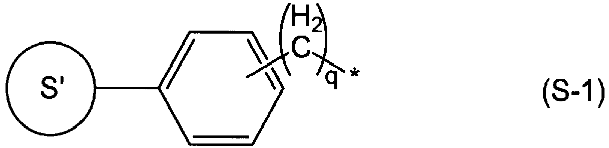 wherein, in the formula (S-1), S' is a part of S, q is 0 or 1, and the asterisk is a binding site to L, 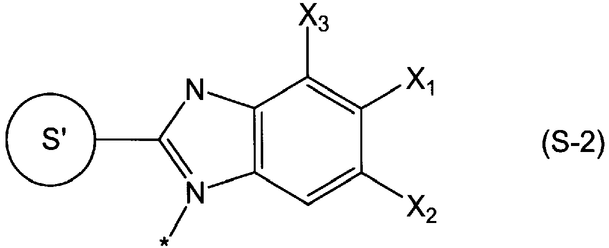 wherein, in the formula (S-2), S' is a part of S, X₁ and X₃ each independently represent a hydrogen atom or a halogen atom, and X₂ represents a hydrogen atom, a halogen atom, or a nitrile group, but at least one of X₁, X₂, and X₃ is a halogen atom, and the asterisk is a binding site to L.

3. The labeling precursor compound according to claim 1, wherein, in the general formulas (1) and (2), S is a group represented by the following formula (S-1) or (S-2): 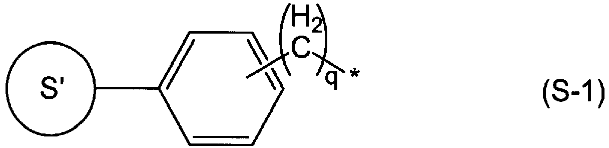 wherein, in the formula (S-1), S' is a part of S, q is 0 or 1, and the asterisk is a binding site to L, 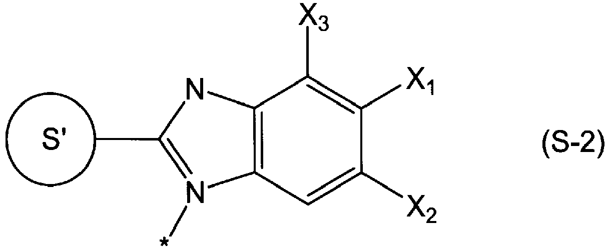 wherein, in the formula (S-2), S' is a part of S, X₁ and X₃ each independently represent a hydrogen atom or a halogen atom, and X₂ represents a hydrogen atom, a halogen atom, or a nitrile group, but at least one of X₁, X₂, and X₃ is a halogen atom, and the asterisk is a binding site to L.

4. A production method of a radioactive fluorine-labeled compound, comprising a step of allowing a labeling precursor compound according to any one of claims 1 to 3 to react with [¹⁸F]fluoride ion to obtain a radioactive fluorine-labeled compound represented by the following general formula (1): 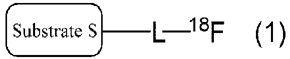 wherein S represents a substrate, and
L represents a straight alkyl group having 1 to 6 carbon atoms, which may contain an ether group.

5. The production method according to claim 4, further comprising:
a step of adding a reaction mixture containing a radioactive fluorine-labeled compound represented by the general formula (1) to a reverse-phase cartridge column, said reaction mixture being obtained by the reaction of the labeling precursor compound with [¹⁸F]fluoride ion; and
a step of eluting the radioactive fluorine-labeled compound represented by the general formula (1) from the reverse-phase cartridge column.

## Patentansprüche

1. Markierungsvorläuferverbindung für eine mit radioaktivem Fluor markierte Verbindung, dargestellt durch die folgende allgemeine Formel (1) 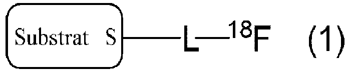
wobei S ein Substrat darstellt, und
L eine gerade Alkylgruppe mit 1 bis 6 Kohlenstoffatomen darstellt, die eine Ethergruppe enthalten kann,
wobei die Markierungsvorläuferverbindung durch die folgende allgemeine Formel (2) dargestellt wird: 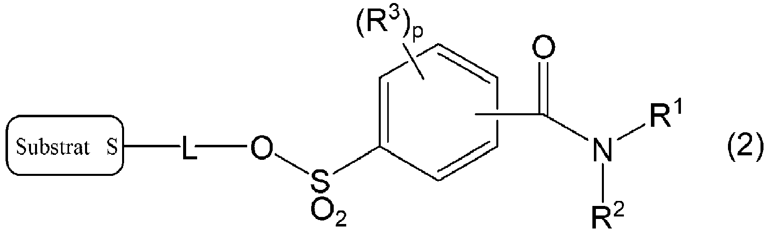
wobei S und L die gleichen wie in der allgemeinen Formel (1) sind,
R¹ und R² jeweils unabhängig voneinander eine gerade oder verzweigte Alkylgruppe mit 1 bis 30 Kohlenstoffatomen oder eine substituierte oder unsubstituierte monocyclische oder kondensierte polycyclische Arylgruppe darstellen,
R³ jeweils unabhängig voneinander eine Alkylgruppe mit 1 bis 4 Kohlenstoffatomen oder eine Alkoxygruppe mit 1 bis 4 Kohlenstoffatomen darstellt, und
p eine ganze Zahl von 0 bis 4 darstellt,
**dadurch gekennzeichnet, dass** die mit radioaktivem Fluor markierte Verbindung, die durch die allgemeine Formel (1) dargestellt wird, einen clogP-Wert von -1,4 bis 5,0 aufweist, und dass der Unterschied im clogP-Wert zwischen der mit radioaktivem Fluor markierten Verbindung, die durch die allgemeine Formel (1) dargestellt wird, und der Vorläuferverbindung, die durch die allgemeine Formel (2) dargestellt wird, 2 oder mehr ist.

2. Markierungsvorläuferverbindung nach Anspruch 1, wobei, in den allgemeinen Formeln (1) und (2), S eine durch die folgende Formel (S-1) oder (S-2) dargestellte Gruppe ist 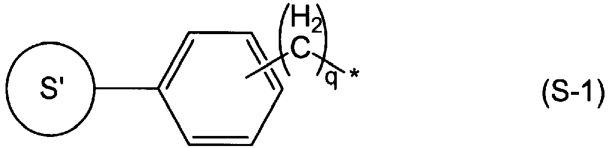 wobei, in der Formel (S-1), S' ein Teil von S ist, q 0 oder 1 ist, und das Sternchen eine Bindungsstelle zu List, 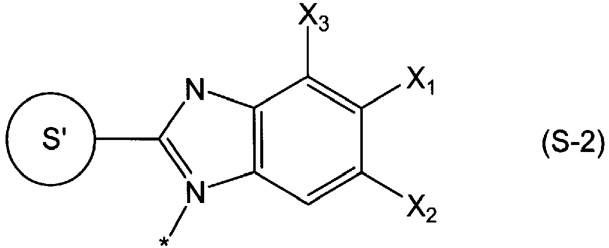 wobei, in der Formel (S-2), S' ein Teil von S ist, X₁ und X₃ jeweils unabhängig voneinander ein Wasserstoffatom oder ein Halogenatom darstellen, und X₂ ein Wasserstoffatom, ein Halogenatom oder eine Nitrilgruppe darstellt, aber mindestens eines von X₁, X₂ und X₃ ein Halogenatom ist, und das Sternchen eine Bindungsstelle für L ist.

3. Markierungsvorläuferverbindung nach Anspruch 1, wobei, in den allgemeinen Formeln (1) und (2), S eine durch die folgende Formel (S-1) oder (S-2) dargestellte Gruppe ist: 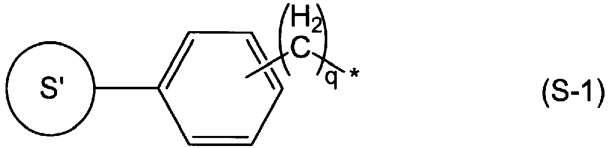 wobei, in der Formel (S-1), S' ein Teil von S ist, q 0 oder 1 ist, und das Sternchen eine Bindungsstelle zu L ist, 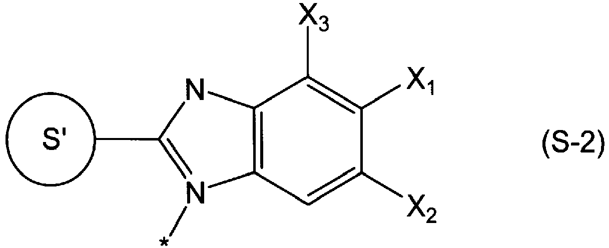 wobei, in der Formel (S-2), S' ein Teil von S ist, X₁ und X₃ jeweils unabhängig voneinander ein Wasserstoffatom oder ein Halogenatom darstellen, und X₂ ein Wasserstoffatom, ein Halogenatom oder eine Nitrilgruppe darstellt, aber mindestens eines von X₁, X₂ und X₃ ein Halogenatom ist, und das Sternchen eine Bindungsstelle für L ist.

4. Verfahren zur Herstellung einer mit radioaktivem Fluor markierten Verbindung, umfassend einen Schritt, bei dem eine Markierungsvorläuferverbindung nach einem der Ansprüche 1 bis 3 mit einem [¹⁸F]Fluoridion reagieren gelassen wird, um eine mit radioaktivem Fluor markierte Verbindung der folgenden allgemeinen Formel (1) zu erhalten: 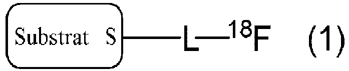 wobei S ein Substrat darstellt, und
L eine geradkettige Alkylgruppe mit 1 bis 6 Kohlenstoffatomen darstellt, die eine Ethergruppe enthalten kann.

5. Herstellungsverfahren nach Anspruch 4, ferner umfassend:
einen Schritt der Zugabe einer Reaktionsmischung, die eine mit radioaktivem Fluor markierte Verbindung der allgemeinen Formel (1) enthält, zu einer Umkehrphasen-Kartuschensäule, wobei die Reaktionsmischung durch die Reaktion der Markierungsvorläuferverbindung mit [¹⁸F]Fluoridionen erhalten wird; und
einen Schritt des Eluierens der durch die allgemeine Formel (1) dargestellten, mit radioaktivem Fluor markierten Verbindung von der Umkehrphasen-Kartuschensäule.

## Revendications

1. Composé précurseur de marquage pour un composé marqué au fluor radioactif représenté par la formule générale (1) suivante : 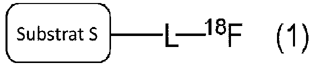 dans laquelle S représente un substrat, et
L représente un groupe alkyle linéaire ayant 1 à 6 atomes de carbone, qui peut contenir un groupe éther,
le composé précurseur de marquage étant représenté par la formule générale (2) suivante : 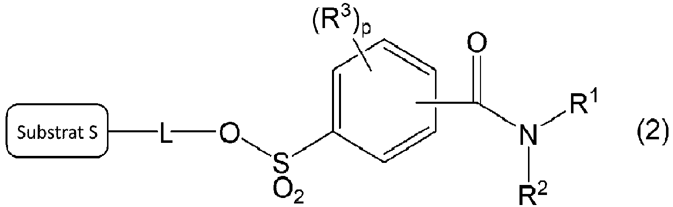
dans laquelle S et L sont tels que dans la formule générale (1),
chacun de R¹ et R² représente indépendamment un groupe alkyle linéaire ou ramifié ayant 1 à 30 atomes de carbone, ou un groupe aryle polycyclique condensé ou monocyclique substitué ou non substitué,
chaque R³ représente indépendamment un groupe alkyle ayant 1 à 4 atomes de carbone ou un groupe alcoxy ayant 1 à 4 atomes de carbone, et
p représente un entier de 0 à 4,
**caractérisé en ce que** le composé marqué au fluor radioactif représenté par la formule générale (1) a une valeur clogP de -1,4 à 5,0, et la différence de clogP entre le composé marqué au fluor radioactif représenté par la formule générale (1) et le composé précurseur représenté par la formule générale (2) est de 2 ou plus.

2. Composé précurseur de marquage selon la revendication 1, dans lequel, dans les formules générales (1) et (2), S est un groupe représenté par l'une des formules (S-1) et (S-2) qui suivent : 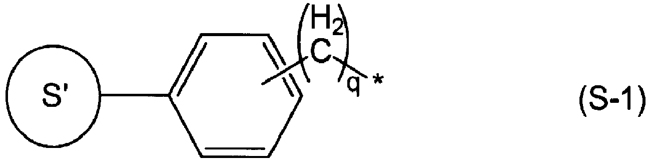 dans lequel, dans la formule (S-1) , S' est une partie de S, q vaut 0 ou 1, et l'astérisque est un site de liaison à L, 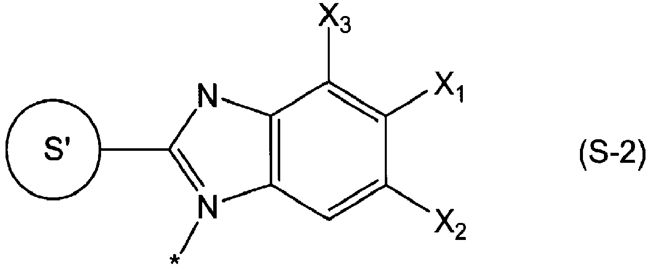 dans lequel, dans la formule (S-2), S' est une partie de S, chacun de X₁ et X₃ représente indépendamment un atome d'hydrogène ou un atome d'halogène, et X₂ représente un atome d'hydrogène, un atome d'halogène, ou un groupe nitrile, mais au moins l'un de X₁, X₂ et X₃ est un atome d'halogène, et l'astérisque est un site de liaison à L.

3. Composé précurseur de marquage selon la revendication 1, dans lequel, dans les formules générales (1) et (2), S est un groupe représenté par l'une des formules (S-1) et (S-2) qui suivent : 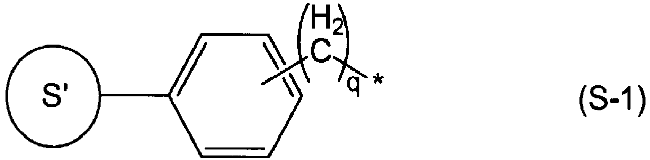 dans lequel, dans la formule (S-1) , S' est une partie de S, q vaut 0 ou 1, et l'astérisque est un site de liaison à L, 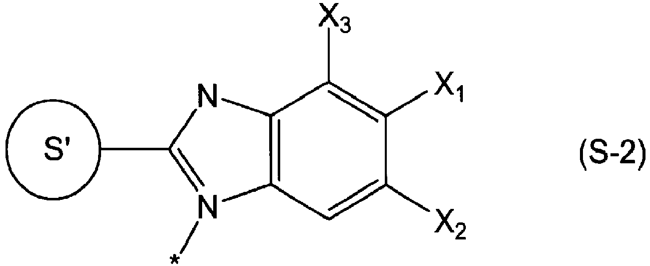 dans lequel, dans la formule (S-2), S' est une partie de S, chacun de X₁ et X₃ représente indépendamment un atome d'hydrogène ou un atome d'halogène, et X₂ représente un atome d'hydrogène, un atome d'halogène, ou un groupe nitrile, mais au moins l'un de X₁, X₂ et X₃ est un atome d'halogène, et l'astérisque est un site de liaison à L.

4. Procédé de production d'un composé marqué au fluor radioactif, comprenant une étape consistant à laisser un composé précurseur de marquage selon l'une quelconque des revendications 1 à 3 réagir avec un ion fluorure [¹⁸F] pour que soit obtenu un composé marqué au fluor radioactif représenté par la formule générale (1) suivante : 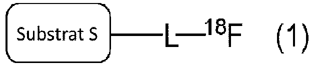 dans laquelle S représente un substrat, et
L représente un groupe alkyle linéaire ayant 1 à 6 atomes de carbone, qui peut contenir un groupe éther.

5. Procédé de production selon la revendication 4, comprenant en outre :
une étape consistant à ajouter un mélange réactionnel contenant un composé marqué au fluor radioactif représenté par la formule générale (1) à une colonne à cartouche à inversion de phases, ledit mélange réactionnel étant obtenu par la réaction du composé précurseur de marquage avec un ion fluorure [¹⁸F] ; et
une étape consistant à éluer le composé marqué au fluor radioactif représenté par la formule générale (1) à partir de la colonne à cartouche à inversion de phases.
